(19) Europäisches Patentamt
European Patent Office
Office européen des brevets

(11) Numéro de publication : **0 097 577**
**B1**

(12) # FASCICULE DE BREVET EUROPÉEN

(45) Date de publication du fascicule du brevet : **02.01.86**

(51) Int. Cl.⁴ : **B 01 D 13/00**, B 01 D 35/00

(21) Numéro de dépôt : **83401197.5**

(22) Date de dépôt : **10.06.83**

(54) **Appareil de filtration rapide, notamment pour liquides biologiques.**

(30) Priorité : **16.06.82 FR 8210498**

(43) Date de publication de la demande : **04.01.84 Bulletin 84/01**

(45) Mention de la délivrance du brevet : **02.01.86 Bulletin 86/01**

(84) Etats contractants désignés : **DE GB**

(56) Documents cités :
**FR-A- 2 161 019**
**FR-A- 2 278 374**
**GB-A- 1 311 565**
**US-A- 3 565 256**

(73) Titulaire : **COMMISSARIAT A L'ENERGIE ATOMIQUE Etablissement de Caractère Scientifique Technique et Industriel**
**31/33, rue de la Fédération**
**F-75015 Paris (FR)**

(72) Inventeur : **Dupont, Yves**
**Les Vernes**
**F-38250 Lans-En-Vercors (FR)**
Inventeur : **Rocher, Christian**
***Route de Saint Georges Champ sur Drac***
**F-38650 Jarrie (FR)**

(74) Mandataire : **Mongrédien, André et al**
**c/o BREVATOME 25, rue de Ponthieu**
**F-75008 Paris (FR)**

**EP 0 097 577 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention concerne un appareil de filtration permettant de réaliser cette opération dans un minimum de temps. Ce dispositif de filtration rapide s'applique plus particulièrement au domaine biologique, notamment à deux types d'études très répandues en biochimie et en bioénergétique : les études de mécanisme enzymatique et les mesures de flux ioniques à travers les membranes cellulaires.

L'étude détaillée des réactions enzymatiques ainsi que celle des mécanismes contrôlant les flux ioniques nécessitent l'élaboration d'outils perfectionnés permettant de résoudre les événements transitoires du processus étudié. Le plus souvent, les réactions catalysées par des enzymes se produisent à très grande vitesse et nécessitent des techniques permettant une résolution temporelle de l'ordre de quelques millisecondes. On constate en fait que les progrès dans notre connaissance des mécanismes de l'action enzymatique ont suivi en général une amélioration de la technique utilisée et en particulier la résolution en temps obtenue. Ainsi, on sait que :

1) la durée d'un cycle enzymatique est en général bien inférieure à la seconde. Dans un cycle peuvent être en général identifiés un assez grand nombre d'états intermédiaires. Des méthodes de mélange rapide (« multi-mixing » techniques) ont été développées dans le passé dans le but d'étudier ces états transitoires. Dans leur principe, ces méthodes sont relativement simples : l'enzyme E est mélangé très rapidement (quelques millisecondes) avec une molécule ou substrat S, la réaction suit ensuite son cours et est achevée lors de la libération du produit P :

$$E + S \rightarrow E \cdot S \rightarrow E \cdot {}^{+}S \rightarrow E^{+} \cdot P \rightarrow E + P \qquad (1)$$

Q (→ $E \cdot {}^{+}S$)

L'analyse des étapes intermédiaires (ici l'état $E \cdot {}^{+}S$) s'effectue en procédant à un second mélange rapide (quelques millisecondes à quelques secondes après le mélange initial) avec un réactif Q stabilisant l'espèce $E^{+}S$. Cette espèce est ensuite analysée par des techniques biochimiques classiques mais pouvant être lentes. L'enzyme est mélangé très rapidement avec la molécule ou substrat avec lequel il doit s'associer avant de produire sa modification.

Le but de la manipulation est d'étudier la réaction (1).

L'enzyme est mis en contact avec la molécule à étudier (ou substrat S).

Dans un second stade il y a association de l'enzyme et de la molécule ou substrat, puis modification de la molécule par l'enzyme.

On bloque à ce stade la réaction à l'aide d'un réactif Q ; on peut donc analyser cette espèce bloquée ($E^{+}S$) par des méthodes biochimiques classiques, comme le mélange rapide ainsi qu'indiqué précédemment.

On peut bloquer la réaction à tous les stades si on dispose d'un réactif approprié ($E^{+}S$, $E^{+}P$, $E + P$). Une autre technique consiste à substituer au substrat naturel S un analogue chimique (molécule ayant les mêmes propriétés chimiques mais des propriétés physiques particulières qui peuvent être suivies par voie optique (fluorescence, absorbance) permettant de suivre les progrès de la réaction (1) en fonction du temps. Le pouvoir de résolution en temps des méthodes de mélange rapide et des méthodes optiques se situe autour de 10 msec.

2) Les mesures de flux ioniques (flux ionique = mesure des flux de molécules, d'ions, ... entrés et sortis dans une molécule) ont été, par le passé, presque exclusivement effectuées en utilisant des méthodes de filtration (ex : filtres « Millipore »). En bref, le principe de ces mesures est le suivant : une suspension de cellules ou de vésicules membranaires est diluée dans un milieu contenant le substrat ou molécule dont on veut étudier le flux ionique devant être transporté dans la cellule. En général ce substrat est marqué par un isotope radioactif. Après un temps d'incubation plus ou moins long, la suspension est soumise à une filtration. Les membranes ou les cellules sont retenues par le filtre et le transport est évalué en mesurant la radioactivité du filtre à l'aide d'un compteur à scintillation. Cette technique est assez lente (minimum deux secondes), mais c'est la seule disponible actuellement. Elle est donc très utilisée car les mesures de flux ont une très grande importance pour les recherches sur la physiologie et l'énergétique des cellules ainsi qu'en pharmacologie moléculaire.

L'appareil faisant l'objet de la présente invention autorise une filtration très rapide, ce qui permet de suivre les différentes étapes des réactions au sein d'un liquide biologique, notamment en biochimie et en bioénergétique.

Dans ce but, l'invention propose un appareil de filtration comportant :

— un dispositif d'injection muni d'un réservoir fermé à sa base par une pastille poreuse et à son sommet par un piston déplaçable en translation dans le réservoir par tout moyen mécanique approprié, ledit réservoir renfermant la phase liquide à filtrer ;

— un dispositif support de filtre comprenant une chambre fermée à sa partie supérieure par une pastille poreuse servant de support à la matière filtrante proprement dite et communiquant par sa base avec des moyens de pompage permettant de créer une dépression dans ladite chambre ;

— un dispositif de mise en contact rapide et temporaire du dispositif d'injection et du dispositif support de filtre ;

— un ensemble électronique de commande qui contrôle chaque opération de filtrage en agissant simultanément sur le dispositif de mise en contact et sur les moyens mécaniques de translation du piston et en provoquant, au moment souhaité, l'arrêt de l'injection et la désolidarisation du dispositif d'injection et du dispositif support du filtre.

Selon un premier mode de réalisation de l'invention, les pastilles poreuses sont en verre fritté.

Selon un deuxième mode de réalisation de l'invention, les moyens mécaniques de déplacement du piston en translation sont constitués par un moteur pas à pas.

Enfin, selon un dernier mode de réalisation de l'invention, le dispositif de mise en contact rapide et temporaire du dispositif d'injection et du dispositif support de filtre se compose d'un solénoïde alimenté électriquement par l'ensemble électronique de commande et actionnant un levier capable de provoquer la translation verticale du support de filtre en le rapprochant ou en l'éloignant du dispositif d'injection.

De toute façon, l'invention sera mieux comprise en se référant à la description qui suit, donnée à titre illustratif et non limitatif.

La figure 1 représente en coupe élévation l'appareil conforme à l'invention tandis que la figure 2 illustre une mesure de la cinétique de liaison des ions $Ca^{++}$ sur une protéine membranaire.

Sur la figure 1, on a représenté le dispositif d'injection 1 qui comporte, maintenu en place sur un support 2 par une vis de maintien 3, un réservoir cylindrique 4 fermé à sa base par une pastille poreuse 5. A sa partie supérieure, le réservoir cylindrique 4 est fermé par un piston 6 déplaçable en translation verticale dans le réservoir cylindrique 4 et dont les déplacements sont commandés par un moteur pas à pas 7 agissant directement sur la tête 8 du piston 6.

L'appareil de filtration rapide, objet de l'invention, comprend également un dispositif support de filtre 9 muni en son centre d'une chambre cylindrique 10 fermée à sa partie supérieure par une autre pastille poreuse 11. La pastille poreuse 11 sert de support à la matière filtrante proprement dite représentée en 12 et qui peut être constituée de façon connue d'un filtre, de gel, de microbilles de verre ou de tout autre support en matière synthétique. Une pompe 13 permet le moment venu de créer une dépression dans la chambre cylindrique 10. Des moyens en guidage en translation 14, d'un type en soi connu, permettent de rendre le dispositif support de filtre 9 mobile en translation axiale dans l'axe du dispositif d'injection 1 sous l'effet du dispositif de mise en contact rapide et temporaire 15. Ce dispositif 15 est susceptible d'agir à distance, par action électromagnétique par exemple, sur un levier 16 mobile autour d'un axe horizontal 17 et dont l'extrémité 18 commande par l'intermédiaire de la rotule 19 le mouvement de translation du dispositif support de filtre 9 le long de son axe. Un ensemble électronique de commande 20 permet d'agir simultanément par la ligne 21 sur le moteur pas à pas 7 et par les lignes 22 et 23 sur le dispositif de mise en contact rapide 15.

Dans la réalisation décrite sur la figure 1, le dispositif de mise en contact rapide et temporaire 15 se compose d'un solénoïde alimenté électriquement par l'ensemble électronique de commande 20 et capable de créer un champ magnétique d'intensité suffisante pour provoquer l'attraction de la palette ferro-magnétique 24 et le basculement du levier 16 autour de son axe 17 comme indiqué par les flèches FF'.

Le fonctionnement de l'appareil de filtration rapide décrit sur la figure unique est le suivant.

La phase liquide que l'on désire filtrer est préalablement introduite dans le réservoir cylindrique 4 du dispositif d'injection 1. La matière de filtration, par exemple un filtre « Millipore », est déposée à la surface de la pastille poreuse 11 et l'appareil est alors prêt à fonctionner. Sous l'effet de la commande électronique 20, le dispositif de mise en contact rapide et temporaire 15 du dispositif d'injection 1 et du support de filtre 9 agit sur le levier 16 qui bascule autour de son axe 17 provoquant le rapprochement rapide à l'aide des moyens de guidage en translation 14 du support de filtre 9 vers le dispositif d'injection 1 qui, lui, reste fixe. Dès que le contact entre les deux parties précédentes de l'appareil est établi, un détecteur non représenté sur la figure transmet l'information de contact à la commande 20 qui agit immédiatement par la ligne 21 sur le moteur pas à pas 7 pour introduire d'une quantité déterminée le piston 6 dans la chambre 4 et provoquer la filtration très rapide d'une partie de cette phase liquide contenue dans 4 au travers des deux pastilles poreuses 5 et 11 et de la matière filtrante à proprement parler 12. En même temps les moyens de pompage 13 sont mis en action par l'ensemble électronique de commande 20 et, par création d'une dépression dans la chambre cylindrique 10, aident à accélérer la filtration ainsi que l'évacuation de la phase liquide ayant traversé le filtre.

Au bout d'un temps prédéterminé ou dès que la quantité de phase liquide voulue a été filtrée au travers de l'appareil de filtration rapide, la commande 20 agit en sens inverse sur le dispositif de mise en contact temporaire 15 pour séparer à nouveau les deux moitiés de l'appareil constituées par le dispositif d'injection 1 et le support de filtre 9. Cette façon de procéder permet, notamment lorsque le produit à filtrer est radioactif, de limiter au strict minimum le temps de contamination du filtre.

A titre d'exemple on va décrire ci-après une application de l'appareil de filtration rapide selon l'invention.

Les protéines ou les vésicules membranaires (fragments de membranes nerveuses ou musculaires) sont déposées sur un filtre millipore qui possède les caractéristiques appropriées pour les retenir après élimination de l'excès d'eau. Le filtre contenant le matériel biologique (membranes ou protéines) est

ensuite rapidement lavé par une solution contenant le substrat radioactif, c'est-à-dire la molécule qui va se lier avec les protéines ou membranes bloquées sur le filtre. La réaction avec la molécule ou substrat et son échange avec le milieu filtrant se poursuit pendant tout le temps de filtration. La filtration est arrêtée au moment voulu et le filtre isolé du réactif, la radioactivité contenue dans le filtre est mesurée et permet de rendre compte de l'état de la réaction au moment de l'arrêt de la filtration. Le dispositif mis au point permet d'ajuster très précisément le temps de passage du liquide ainsi que le volume filtré. Il a été possible d'obtenir des temps de filtration aussi courts que quelques dizaines de millisecondes, c'est-à-dire une résolution temporelle inférieure de deux ordres de grandeur au moins à celle obtenue avec les méthodes de filtration usuelles (2-5 sec. au moins).

Cette caractéristique fait de cet appareil un outil extrêmement intéressant pour les mesures de transport ionique à travers les membranes cellulaires. Cela permet également d'envisager des mesures de cinétiques enzymatiques tout à fait originales, en particulier cet appareil permet d'aborder l'étude des complexes enzymes-substrat ou molécules non covalents. Une telle mesure est décrite en détail ci-après se rapportant à une mesure de la cinétique de liaison d'ions $Ca^{++}$ sur une protéine membranaire (ATPase-$Ca^{++}$ du Reticulum Sarcoplasmique).

Chaque mesure est effectuée comme suit :

— un filtre « Millipore » (HAWP 0,45 μm) est déposé sur le support de filtre,

— les vésicules membranaires contenant la protéine sont déposées sur le filtre (200 à 300 μg de protéines par filtre),

— le support de filtre est placé sous le dispositif d'injection rempli d'une solution contenant du $^{45}Ca$,

— la réaction est déclenchée par le système électronique de contrôle qui met en contact le filtre et le dispositif d'injection pendant un temps prédéterminé (20 msec à plusieurs secondes). Pendant ce temps, le dispositif d'injection est actionné et la solution contenant le $^{45}Ca$ est injectée à travers le filtre (0,2 à 1 ml).

La quantité de liquide filtré est également prédéterminée.

— la réaction et la filtration se terminent par la séparation physique du filtre et du dispositif d'injection,

— la radioactivité du filtre est mesurée à l'aide d'un compteur à scintillation,

— cette séquence est répétée pour chaque temps de filtration désiré et le résultat obtenu est la cinétique de liaison montrée sur la figure 2 ci-après, montrant la quantité de $Ca^{2+}$ fixée par les vésicules membranaires en fonction du temps de contact de ces dernières avec la solution de $^{45}Ca^{2+}$. Chaque point (0) (courbe I) correspond à une mesure différente et donc chaque fois à un nouveau filtre contenant la protéine à étudier. Les points (O) (courbe II) correspondent à des mesures effectuées avec des filtres ne contenant pas de protéine et correspondent donc à une mesure de la radioactivité piégée dans l'eau de mouillage du filtre.

La cinétique de liaison des ions $Ca^{2+}$ obtenue dans cette série d'expériences est ensuite analysée et permet de vérifier certains détails de fonctionnement de cette protéine.

## Revendications

1. Appareil de filtration rapide notamment pour liquides biologiques comprenant en combinaison :

— un dispositif d'injection (1) muni d'un réservoir (4) fermé à sa base par une pastille poreuse (5) et à son sommet par un piston (6) déplaçable en translation dans le réservoir (4) par tout moyen mécanique approprié, ledit réservoir (4) renfermant la phase liquide à filtrer ;

— un dispositif support de filtre (9) comprenant une chambre (10) fermée à sa partie supérieure par une pastille poreuse (11) servant de support à la matière filtrante proprement dite (12) et communiquant par sa base avec des moyens de pompage (13) permettant de créer une dépression dans ladite chambre (10) ;

— un dispositif (15) de mise en contact rapide et temporaire du dispositif d'injection (1) et du dispositif support de filtre (9) ;

— un ensemble électronique de commande (20) qui contrôle chaque opération de filtrage en agissant simultanément sur le dispositif de mise en contact (15) et sur les moyens mécaniques (7) de translation du piston (6) et en provoquant, au moment souhaité, l'arrêt de l'injection et la désolidarisation du dispositif d'injection (1) et du dispositif support du filtre (9).

2. Appareil de filtration selon la revendication 1, caractérisé en ce que les pastilles poreuses (5, 11) sont en verre fritté.

3. Appareil de filtration selon la revendication 1, caractérisé en ce que les moyens mécaniques de déplacement du piston (6) en translation sont constitués par un moteur pas à pas (7).

4. Appareil de filtration selon la revendication 1, caractérisé en ce que le dispositif (15) de mise en contact rapide et temporaire du dispositif d'injection (1) et du dispositif support de filtre (9) se compose d'un solénoïde alimenté électriquement par l'ensemble électronique de commande (20) et actionnant un levier (16) capable de provoquer la translation verticale du support de filtre (9) en le rapprochant ou en l'éloignant du dispositif d'injection (1).

**Claims**

1. Rapid filtration apparatus, especially for biological liquids comprising in combination :

— an injection device (1) having a reservoir (4) closes at its base by a porous plate (5) and at its top by a piston (6) movable through said reservoir (4) by any convenient mechanical means, said reservoir (4) enclosing the liquid phase to be filtered ;

— a filter support device (9) comprising a chamber (10) closed at its top by a porous plate (11) acting as support for filtered material (12) and communicating at its base with pump means (13) producing a pressure reduction within said chamber (10) ;

— a device (15) for rapidly and temporarily bringing into contact the injection device (1) and the filter support device (9) ; and

— an electronic command assembly (20) controlling each filtering operation and acting simultaneously on the contracting device (15) and the mechanical moving means (7) for the piston (6) and producing, at the required time, halting of the injection and separation of the injection device (1) and the filter support device (9).

2. Filtration apparatus according to claim 1, characterised in that the porous plates (5, 11) are glass frits.

3. Filtration apparatus according to claim 1, characterised in that the mechanical piston-displacement means (6) comprise a step motor (7).

4. Filtration apparatus according to claim 1, characterised in that the device (15) for rapidly and temporarily contacting the injection device (1) and the filter support device (9) comprise a solenoid supplied with electricity by the electronic command assembly (20) and actuating a lever (16) adapted to produce vertical translation of the filter support (9) towards and away from the injection device (1).

**Patentansprüche**

1. Vorrichtung zur schnellen Filtrierung, besonders von biologischen Flüssigkeiten, die in Kombination umfaßt :

— eine Einspritzvorrichtung (1), die mit einem Behälter (4) ausgerüstet ist, der an seiner Basis mit einer porösen Tablette (5) und oben mit einem Kolben (6) verschlossen ist, welcher durch jedes geeignete mechanische Mittel in dem Behälter (4) verschiebbar ist, wobei der Behälter (4) die zu filtrierende flüssige Phase einschließt,

— eine Filterhaltevorrichtung (9) mit einer Kammer (10), die an ihrem oberen Abschnitt durch eine poröse Tablette (11) geschlossen ist, die als Stütze für die eigentliche gefilterte Materie (12) dient, und die mit ihrer Basis mit Pumpmitteln (13) verbunden ist, mit denen ein Unterdruck in der Kammer (10) erzeugbar ist,

— eine Vorrichtung (15) zum schnellen und vorübergehenden Inberührungbringen der Einspritzvorrichtung (1) und der Filterhaltevorrichtung (9),

— eine elektronische Steuereinheit (20), die jeden Filtervorgang steuert, indem sie gleichzeitig auf die Vorrichtung (15) zum Inberührungsbringen und die mechanischen Mittel (7) zur Verschiebung des Kolbens (6) wirkt und zum erwünschten Zeitpunkt das Anhalten des Einspritzens und die Trennung zwischen Einspritzvorrichtung (1) und Filterhaltevorrichtung (9) hervorruft.

2. Vorrichtung zur Filterung nach Anspruch 1, dadurch gekennzeichnet, daß die porösen Tabletten (5, 11) aus Frittierglas sind.

3. Vorrichtung zum Filtrieren nach Anspruch 1, dadurch gekennzeichnet, daß die mechanischen Mittel zur Verschiebung des Kolbens (6) von einem Schrittmotor (7) gebildet sind.

4. Vorrichtung zum Filtrieren nach Anspruch 1, dadurch gekennzeichnet, daß die Vorrichtung (15) zum schnellen und vorübergehenden Inberührungbringen der Einspritzvorrichtung (1) und der Filterhaltevorrichtung (9) aus einem Solenoid besteht, der elektrisch von der elektronischen Steuereinheit (20) versorgt wird und einen Hebel (16) betätigt, mit dem die vertikale Verschiebung der Filterhalterung (9) hervorrufbar ist, indem diese der Einspritzvorrichtung (1) genähert oder von ihr entfernt wird.

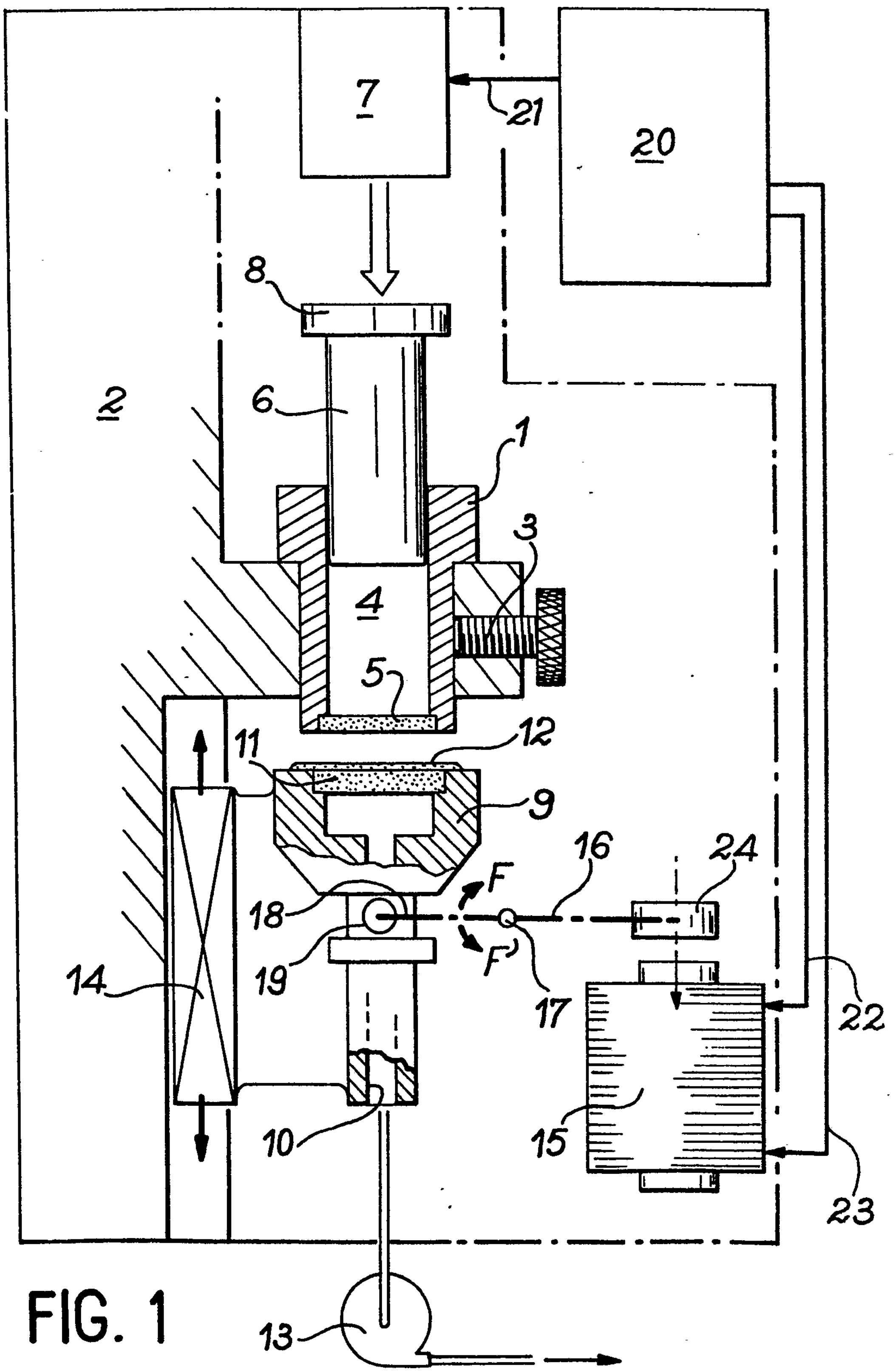


FIG. 1

FIG. 2

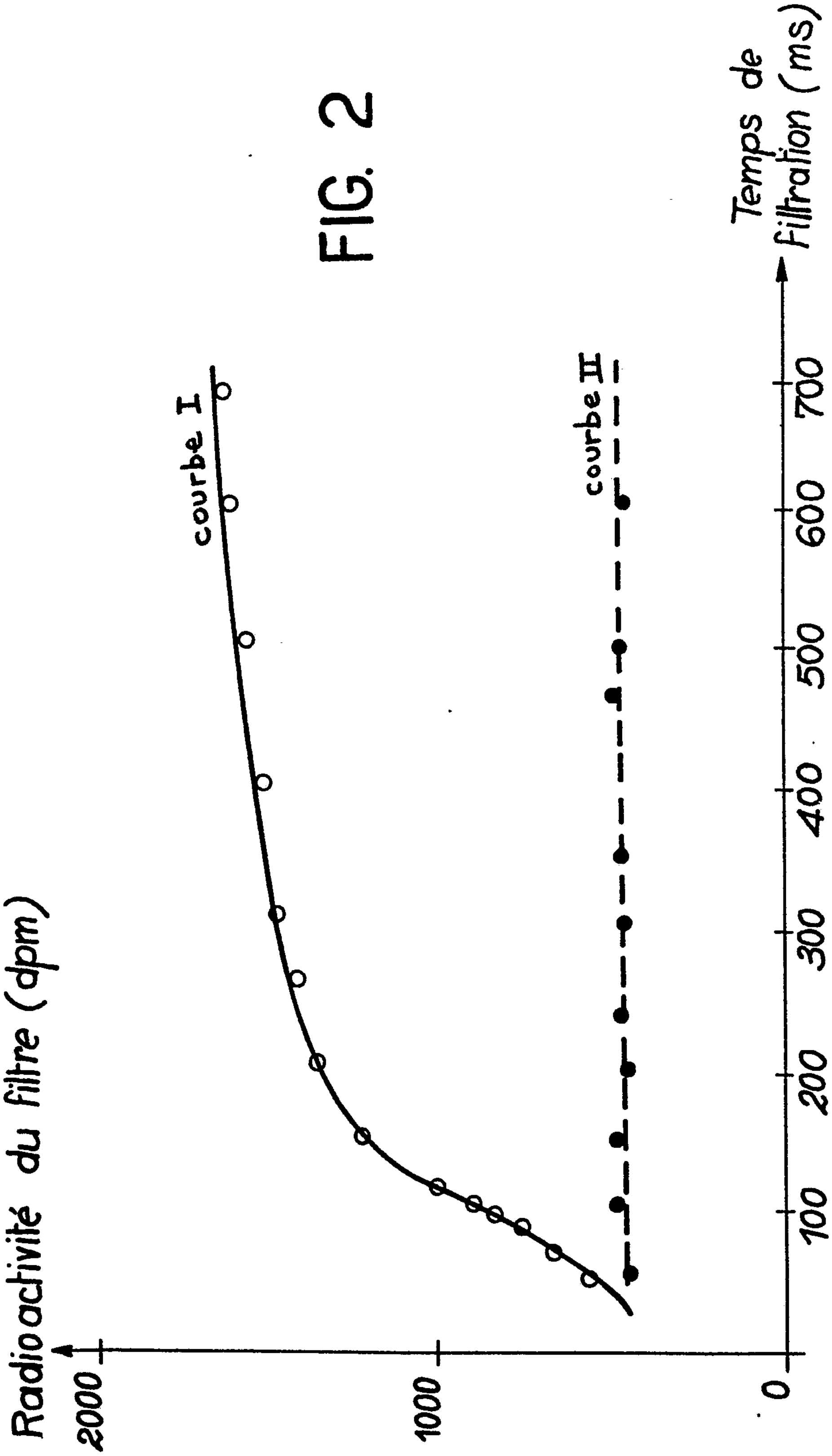
Radioactivité du filtre (dpm)
2000
1000
0
courbe I
courbe II
100
200
300
400
500
600
700
Temps de Filtration (ms)